# EUROPEAN PATENT APPLICATION

(11) **EP 4 765 139 A1**
(43) Date of publication of application: **24.06.2026**
(21) Application number: 24223019.1
(22) Date of filing: 23.12.2024
(51) Int. Cl.: G16H 10/60, G16H 30/20

(54) **OPERATING ROOM DATA CONTROL AND COMMUNICATION SYSTEM**

(71) Applicant: Baxter Medical Systems GmbH + Co. KG, 07318 Saalfeld (DE)
(72) Inventor: FERRARI, Ambra, 07318 Saalfeld (DE); SALVATERRA, Davide, 07318 Saalfeld (DE); CONTESSI, Paolo, 07318 Saalfeld (DE); MAFFEI, Raffaele, 07318 Saalfeld (DE)
(74) Representative: Reddie & Grose LLP

(57) **Abstract**

An operating room data system and method is provided for control and communication of data. The operating room data system comprises one or more adaptors; and a communication unit. The one or more adaptors are configured to receive two or more data sets, each data set received from a source device and having a different data format; convert the data format of each data set to a unified format; and send the converted data set to the communication unit for data storage.

## Description

### TECHNICAL FIELD

This disclosure relates to a system and method for operating room data control and communication. In particular, the disclosure relates to conversion, storage and access of data sets received from devices such as medical devices of an operating room.

### BACKGROUND

Technology currently carries an essential role in any up-to-date operating theatre or operating room (OR); lately, the aim to better both the working conditions in the OR and the patient's safety, have attracted a large amount of interest and technological development. Current ORs are filled with extremely specialized high tech apparatus and devices.

In particular, current surgery has grown dependent to video and audio systems thanks to the recent advent of robotics, along with the introduction of minimal invasive surgery, and interventional radiology.

The technology is complex and requires efficiency and organization. Each apparatus present in the OR covers a specific task, carries its specific monitor, holds its own cabling, along with a command console; each device is moved around the OR according to the needs of the surgery. The presence of high-tech tools in the OR leads to an increase in complexity within the OR environment, requiring the medical team, and the surgeon in particular, to interact with and control increasingly sophisticated medical equipment, which may not be easy, particularly in sterile environments. Further, the complexity of monitoring and tracking objects within the OR, such as particular medical devices, or patient position or vital signs, increases as the number and complexity of the medical devices within the OR increases.

Further, the addition of state of the art video and audio technology, as well as monitoring technology (heart rate, respiratory information etc.) within the OR means that large amounts of data are collected from the OR. However, the type and format of this data may vary greatly due to the large number of different data sources from within the OR. For example, there may be multiple sources of audio, video and or image data, metadata relating to each source, multiple sources of patient tracking data and associated metadata, each with its own format set by the source collecting the data. In such a complex environment, it may be challenging to efficiently route, store and access this data.

An important task of the overall OR System is to provide an environment which allows for effective data collection, storage and access.

However, previous OR Integrated Systems do not allow for flexible and dynamic data routing and communication from devices inside the OR to the outside world. Further, storage and management of a given data set is often tied to the data format being employed by the source device. However, in an environment with multiple different source devices collecting data sets in difference formats, the management of these different data sets may be complex and inefficient.

Accordingly, there is still a need to provide a OR systems and methods which solves any or all of the disadvantages of the known approaches described above.

### SUMMARY

The systems and methods disclosed herein improve upon the existing data control systems for operating rooms and accordingly have provided an disclosure , embodiments of which may have benefits including improved data collection and storage, complexity reduction and improvements in efficiency, particularly in relation to different source devices operating according to different formats and a centralized data management system.

The systems and methods according to the disclosure are defined in the appended claims, to which reference is now directed. Preferred features are set out in the dependent claims.

An operating room data system for a medical facility is provided according to a first aspect of this disclosure. The system comprises one or more adaptors; and a communication unit. The one or more adaptors are configured to: receive two or more data sets, each data set received from a source device and having a different data format; convert the data format of each data set to a unified format; and send the converted data sets to the communication unit for data storage.

Thus, a system is provided that is able to dynamically receive data sets of different formats from source devices (e.g. multiple different source devices such as medical devices with recording functionality, cameras, microphones, imaging devices, CT, MRI, OR Integration Systems etc.) and convert all the data into a unified format (that is, a unified data formatting scheme the system uses for centralised processing and management of the data) for storage. Thus, rather than a data management system for an OR being limited to source devices having a single format, which would limit the possible source devices from which an OR could collect data, or data being collected from the various sources independently and in an uncoordinated fashion, and then stored and manged by separate data management systems in their source formats, a centralised data system is provided that is agnostic as to the formats of the various source devices in the OR. The system is able to interface with source devices of any format, centralise and coordinate reception of multiple data sets from the OR, convert the sets into a unified format and then store the converted data. This has a number of advantages. For example, it allows a greater flexibility in the source devices an OR can utilise while maintaining a centralised data management function; any source device can be used regardless of its native data format. In addition, by dynamically converting into a unified format data from multiple different source devices as they are being received from an OR allows immediate centralisation of storage. Different data sets of different formats are not stored in separate datastores according to type and format, but rather may be stored in the same storage location (e.g. in one or more memory units) and in the same format. This improves efficiency and reduces complexity of storing OR originating data. For example, storage WRITE requests may be unified and centralised. It may also reduce overall storage requirements in view of the system not having to operate with multiple different formats. This also improves coordination of data as the central reception and conversion of all data into a unified format on a common platform facilitates data grouping and oversight, helping to ensure metadata is appropriately linked and stored in relation to the media data or patient that metadata is describing. This system also improves user access to the stored data, as all data becomes accessible to the user through the single unified format (e.g. by way of a user interface a user can navigate to access the data).

Optionally, the communication unit is configured to send the converted data sets to one or more memory units for storage.

Herein, the term "data set" is used broadly to mean a group of associated data of the same general type and the same format. For example, at least one of the two or more data sets may be a media data set comprising one or more of image data, video data and audio data collected by a respective source device from which the data set is received. A media data set may be a data set comprising one or more frames of video data of a surgical procedure collected by an OR room camera or medical device, for example. At least one other of the two or more data sets may be a metadata set comprising metadata (e.g. patient metadata, metadata of a medical procedure, etc.) having a data format different from the format of the media data set. The data is collected by one or more source devices which may communicate the data to the one or more adaptors in one or more data streams, which is to say that an adaptor may receive a data set by receiving a data stream. Depending on the source device, a data stream may comprise one or more data sets of the same type and format (for example, a data stream may comprise one data set representing consecutive frames of a video of a medical procedure collected by an OR camera or medical device, or may comprise multiple consecutive data sets comprising consecutive audio-video files, frames etc., relating to the same or different medical procedures etc.) and the adaptor receiving the stream may convert data as it is received in the stream. A data stream may also comprise data sets of different types and formats, e.g. because a source device collects data of different types and formats. For example, a stream may include frames of video along with associated metadata, both collected by a medical device, in which case an adaptor may receive a single stream comprising a media data set (e.g. an audio-video file) and a corresponding metadata set. Alternatively, in cases where a source device only collects data of a single type (media data or metadata) and format, a data stream may only comprise data of the one type and format. In this case, the one or more adaptors may receive separate streams for the data sets of different types and formats.

It will be appreciated that variations are possible. A source device may collect media data sets of a single format, or may collect media data sets each having different formats. In the latter case, the different media data sets may be communicated to the one or more adaptors in a single data stream or separate data streams. A metadata set may be collected by a source device and sent to the one or more adaptors in a data stream, for example a metadata stream. Like with media data, a source device may collect metadata sets of a single format, or may collect metadata sets each having a different format, in which case the different metadata sets may be communicated to the one or more adaptors in a single data stream or separate data streams. Similarly, a metadata set and corresponding media data set may be collected by a single source device or by separate source devices. In the case of a source device collecting both media data and metadata, the media data set and the metadata set may be sent to the one or more adaptors in a single data stream that comprises both the media data and the metadata, as discussed above. Alternatively, the single source device may send the metadata set and media data set in separate metadata and media data streams. Some source devices may collect both metadata sets and media data sets and communicate both types of data sets to the one or more adaptors (e.g. in a single stream or separate streams). Some source devices may individually collect multiple different sets of media data each having different formats and/or multiple different sets of metadata each having different formats. Some source devices may only collect media data of one format or metadata of one format. The system may involve any combination of such source devices and may utilise the appropriate adaptors and communication pathways in order to receive the various data streams and convert the received media data and metadata into the unified format.

It will be appreciated that any number of media data sets of different formats may be received, for example, 2, 3, 4, 5 or more, each being converted into a unified format. For example, 2, 3 4, 5 or more sets of video data may be received from a corresponding number of different medical devices, each set having a different source format and being converted into a unified format for the system. As with the media data sets, it will be appreciated that any number of metadata sets may be received, for example, 2, 3, 4, 5 or more each set having a different source format and being converted into a unified format for the system. As an example, at least two of the two or more data sets may be video data sets comprising video data, each video data set having a different source video format, wherein the unified format comprises a single video format to which the at least two video data sets are converted, and optionally, the two or more data sets may further comprise one or more metadata sets each of a different data format than the different source video formats, wherein the unified format further comprises a single metadata format to which the one or more metadata sets are converted, the single metadata format being different than the single video format. It will be appreciated that, because the source format may vary from stream to stream (or data set to data set), the conversion of a particular data set to the unified format will differ according to the source format. For example, in the case noted above, one of the at least two video data sets has a first source format, and the conversion into the unified format involves a first conversion (i.e. a conversion from first source format to the unified format), and the other of the at least two video data sets has a second source format, and the conversion into the unified format involves a second conversion (i.e. a conversion from second source format to the unified format). The same is the case for any other different source format, for example the different source formats of the one or more metadata sets. Each different conversion may be performed by a separate adaptor, or an adaptor may perform more than one conversion. A number of possible formats are listed below, but it will be appreciated that any appropriate source data format may be utilised by a given adaptor as a use case necessitates.

As noted above and discussed further below, the "unified format" referrers to a formatting scheme used by the system to centrally manage all data, and may specify or define a single specific format for each of the video, audio, image and metadata, regardless of the source format of the received data set. For example, the formatting scheme may specify a single video format for video data such that any video data received from a source device is converted into that single video format regardless of the source format. The formatting scheme may similarly specify single formats for each of audio data, image data, and metadata. Thus, the data sets are converted to the "unified format" in the sense that they are converted according to a unified formatting scheme of the system.

Optionally, the one or more adaptors are further configured to receive a metadata set for a corresponding media data set of the two or more data sets. The metadata set and the corresponding media data set may be associated with a common identifier, ID, in storage. The one or more adaptors may be configured to attach the common ID to each of the metadata set and the corresponding media data set before sending the metadata set and the corresponding media data set to the communication unit for storage. The metadata set and its corresponding media data set may be related to the same patient, the same operating room and/or the same medical procedure. The metadata set may comprise one or more of image, video, audio, source device, medical procedure or patient metadata. Optionally, the communication unit is configured to send the converted media data sets to the same memory unit and each converted metadata set to one or more memory units different to the memory unit of the converted data sets. In some examples, a corresponding metadata set is received for each received media data set. A data set collected by a source device may be sent to the one or more adaptors in a data stream, for example a media data set may be sent to an adaptor in a media data stream, i.e. a stream of video, audio and/or image data.

The above allows the data management system to be agnostic to data formats for media data sets such as picture or video data as well as metadata sets. The system is able to receive both types of data, convert the data into a unified format and then coordinate and centralize data storage. This allows the media data and its related meta data (for example patient data such as medical history; previously collected health data such as vital signs, blood pressure etc.; information about the medical procedure such as the surgery being performed, timings etc.; information about the source device itself such as device model; information about the media data being sent, etc.) to be stored in a coordinated fashion, which improves identification of relevant metadata.

The communication unit may be configured to provide stored data in the unified format to one or more user devices, for example by receiving from a user device a request for stored data and responsive to receiving the request, routing the requested stored data to the user device. The data may be provided by the communication unit communicating with a user interface that allows requests to be made for stored, for example through review, edit and/or download functions provided at the user interface. Having received a request, data access units of the communication unit may access to one or more memory units to retrieve the requested data, which may then be sent by the communication unit to the user. The communication unit may be further configured to: send stored data to a remote database via a hospital network, e.g. for long term storage. The communication unit may provide one or more user devices data including both stored media data and stored metadata, for example by receiving from a user device a request for stored data and stored metadata for the requested stored data and responsive to receiving the request, routing the requested stored data and metadata to the user device.

Here, the coordinating function of the system is highlighted. The system may centrally provide access to all stored data in a single format. That access may be for any stored data and/or metadata collected from the OR in whatever format, and the system can seamlessly provide the data to the user because the data is already available in a unified format. As the system sits as a central hub between the source devices and end user devices, the system is able to receive all data sets from the OR, convert the data into a unified format and then distribute the data to users appropriately. The system can seamlessly provide the data to the user, with no consideration needed of format; this is handled upset at the point the data is received from, e.g. medical devices. This greatly improves efficiency within the operating room in terms of data collection and management, but also user experience.

Optionally, the communication unit comprises one or more memory units and one or more data access units for retrieving stored data from the one or more memory units (the retrieved data may then be provided by the communication unit to one or more user devices). Further, the one or more adaptors may comprise one adaptor for each received set, or one adaptor may be configured to receive multiple data sets. Further optionally, providing the data to user devices may be over a hospital network. Optionally, at least one source device is located in an operating room. In the case of multiple source devices, each source device may be located in the operating room, or one or more source devices may be located in the operating room and one or more other source devices may be located outside the operating room. Such source devices that are external to the operating room may, for example, be generic IT systems, computers, etc., that are configured to provide data or metadata to the data management system. Such external data may be used to supplement the data being collected from within the OR.

According to a further aspect of the present disclosure, a method of adapting operating room data, the method comprising: receiving two or more data sets, each data set received from a source device and having a different data format; converting the data format of each data set to a unified format; and sending the converted data sets for data storage.

The method may further comprise sending the converted data sets to one or more memory units for storage. At least one of the two or more data sets may be a media data set comprising one or more of image data, video data and audio data collected by a respective source device from which the data set is received. At least one other of the two or more data sets may be a metadata set comprising metadata having a data format different from the format of the media data set. The method may further comprise receiving a metadata set for a corresponding media data set of the two or more data set. The metadata set and the corresponding media data set may be associated with a common ID in storage. The method may further comprise attaching the common ID to each of the metadata set and the corresponding media data set before sending the metadata set and the corresponding media data set to the communication unit for storage. The metadata set and its corresponding media data set may be related to the same patient, the same operating room or the same medical procedure. The metadata set may comprise one or more of image, video, audio, source device, medical procedure or patient metadata. The method may further comprise sending the converted media data set to the same memory unit and each converted metadata set to one or more memory units different to the memory unit of the converted media data set. The method may further comprise providing stored data in the unified format to one or more user devices. The converted data may be sent for storage by a communication unit. The one or more memory units and/or one or more data access units for retrieving stored data from the one or more memory units may be comprised in a communication unit. One or more adaptors may receive the data sets, for example, each data set may be received by a separate adaptor, or multiple data sets may be received by a single adaptor. The one or mor adaptors may be configured to convert each received data set into the unified format. Each source device may be located in the operating room, or one or more source devices may be located in the operating room and one or more other source devices may be located outside the operating room. At least two of the two or more data sets may be video data sets comprising video data, each video data set having a different source video format, wherein the unified format comprises a single video format to which the at least two video data sets are converted, and optionally, the two or more data sets may further comprise one or more metadata sets each of a different data format than the different source video formats, wherein the unified format further comprises a single metadata format to which the one or more metadata sets are converted, the single metadata format being different than the single video format.

According to a still further aspect of the present disclosure, a computer-readable medium is provided having executable instructions stored thereon that when executed, causes any of the system described above to carry out any of the methods described above.

Where functional modules or units are referred to in apparatus embodiments for carrying out various functions, or steps of the described method(s), it will be understood that these modules or units may be implemented in hardware, in software, or a combination of the two. When implemented in hardware, the modules may be implemented as one or more hardware modules, such as one or more application specific integrated circuits (ASICs), or field programmable gate arrays (FPGAs). When implemented in software, the modules may be implemented as one or more computer programs that are executed on one or more processors.

### BRIEF DESCRIPTION OF THE DRAWINGS

Embodiments of the disclosure will be described in more detail by way of example, with reference to the accompanying drawings, in which:
Fig. 1 is a schematic representation of a data control system according to a first embodiment;
Fig. 2 is a schematic representation of the data control system according to the first embodiment, showing various units of the data control system according to a first embodiment;
Fig. 3 is a flow diagram for a data control process according to an embodiment;
Fig. 4 is a flow diagram for a data control process according to aspects of the embodiment; and
Fig. 5 is a flow diagram for a data control process according to aspects of the embodiment.

### DETAILED DESCRIPTION OF THE DISCLOSURE

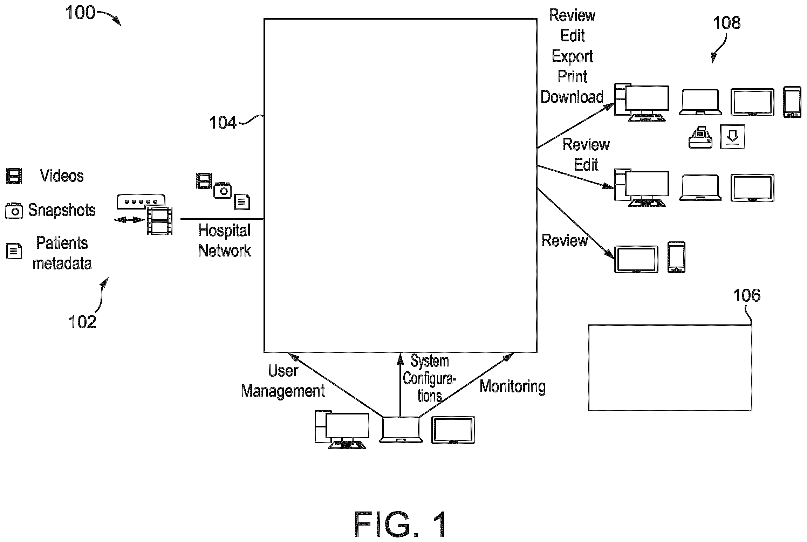

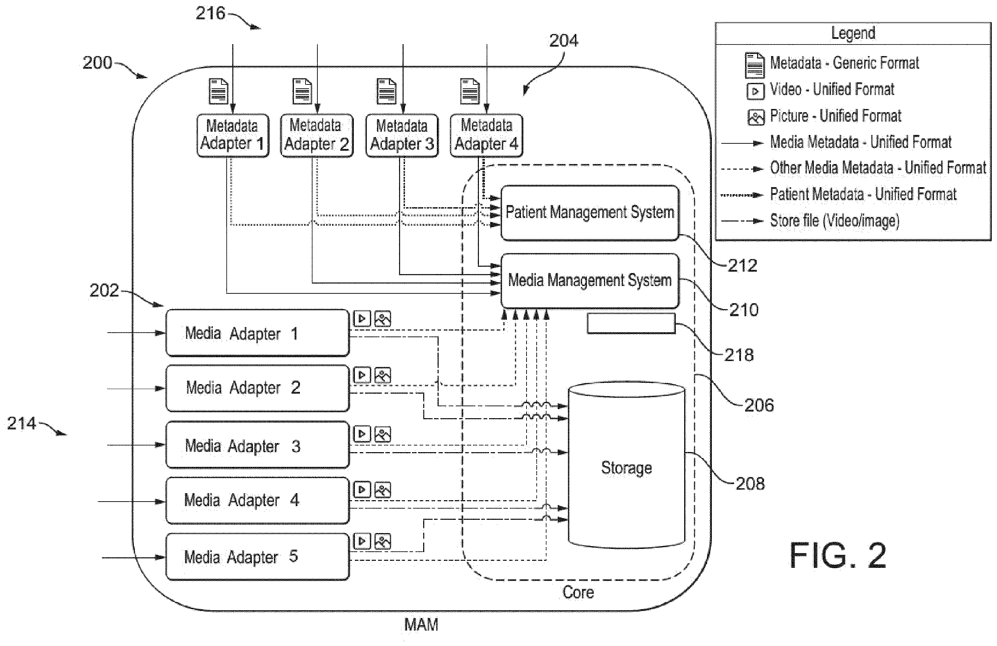

The present disclosure will first present a system overview of an exemplary OR data system which may be used to implement the aspects of the present disclosure. We will then discuss in further detail core components of the overall data system. For ease of discussion, the term OR data system is interchangeable with the term operating room (OR) data control system, hospital data system, or hospital data control system. Whilst the exemplary OR data systems discussed below in relation to figure 1 has a number of components, the principles of the present disclosure are not limited to systems comprising all of these components. For example, an OR data system according to the principles of this disclosure may comprise one or more of the components shown in figure 1. In its broadest implementation, an OR data control system may correspond to the OR control systems of the aspects discussed above in preceding sections, that is comprising one or more adaptors for receiving and converting the data sets from source devices, and a communication unit that may perform any necessary processing, storage management and data access functions. As one example, in the embodiment of figure 1, the OR data control system comprises a number of units, user devices, databases connected together by a hospital network. However, in other embodiments, the OR data control system may only comprise the components shown in figure 2, that is the core unit and the various adaptors, with this OR data control system connecting with user devices and remote databases on a hospital network.

### SYSTEM OVERVIEW

Figure 1 shows an exemplary operating room (OR) data control system 100 comprising one or more data source devices 102, which may be medical devices from one or more ORs, OR 1 to OR n. The data source devices 102 are devices that may be located in the operating room which provide image, video and/or audio data, but it will be appreciated that one or more of the source devices may not be in the same OR or may not be in an OR at all, for example, in the case that data, for example metadata, is being provided by an IT system, computer, etc., external to an OR. Source devices as used herein include devices able to record a video and take pictures, usually from a medical or non medical video device located in the OR. These devices may include any one of an x-ray device, an ultrasound device or other medical eco device, an MRI device, an endoscopy device, a medical monitoring device, a surgical camera, a room camera, etc. These devices are commonly referred to as Video Sources. The OR data system 100 further comprises a hospital data centre 104. The hospital data centre 104 may be referred to herein as a data management system or as a media asset management system (MAM). The OR data system 100 further comprises a remote database 106 such as Electronic Medical Records (EMR), Hospital Information System (HIS), Radiology Information System (RIS) or Picture Archiving and Communication System (PACS), and user devices 108, such as laptops, desktops, tablets, mobile devices, etc. In certain cases, the MAM may send data to the remote database 106 for, e.g. long term storage.

The data sources 102 are connected to the hospital data centre 104 and send data sets, such as image, video and/or audio sets, collected by medical devices or other recording devices within the ORs, to the hospital data centre 104. The hospital data centre 104 centralizes and coordinates the storage of the received data and any associated metadata, including converting the data and metadata into a unified format for storage.

In what follows, the term "unified format" is used broadly to mean the overall data formatting scheme by which the hospital data centre 104 operates. This "unified format" of the system is a formatting scheme that specifies how the system handles the various types of data. It may therefore comprise different specific formats to handle different types of data, but nonetheless together provide the form in which a particular data set must be arranged in order for the data to be placed within a common database of the system for central management, storage, access etc. by the system. For example, for video data, source data may be converted according to the H.264 codec (or other suitable video codec), whereas images may be converted to JPEGs. Audio data may be converted to any suitable format for H.264 video, for example. Therefore, the "unified format" of the system comprises H.264 video and JPEG images; the unified format defines for the system as a whole that any video is according to H.264 and images are JPEGs (in this example). Therefore, conversion of all source video to H.264 and all source images to JPEG is understood herein as a conversion to a "unified" format of the system in the sense of being a conversion that allows the data to fit within the overall database maintained by the hospital data centre 104. The conversion is such that all data (regardless of its original source format) can be managed, stored and accessed centrally by the system.

Data having a format other than the "unified format" therefore means that the data must be modified, "converted", in some manner so as to be translated into the form defined by the unified format, the internal operating form for the system. This means that the specifics of how an adaptor "converts" data for placement in the database may vary according to the type of data. For example, for video data, the conversion may comprise a compression to H.264. In certain examples, the system may allow storage and access of video segments or fragments, and here the conversion of a video data set will include the suitable video fragmentation, storage and handling, according to CMAF. For image data, the conversion may involve a conversion of the source format into JPEG. For metadata, the conversion involves the placement of the metadata into a suitable order and placement for the database, which is to say to specify an order and/or placement appropriate for the database. Thus, the term "convert" or "conversion" is used herein broadly to mean the processing of the source data (media or metadata) so as to put it into a form suitable for centralised management and processing by the system.

Once the one or more adaptors have performed the conversion, the data sets are sent to the communication unit (a part of hospital data centre 104) for storage. The data may be stored centrally and/or sent to remote databases 106 via connection on the hospital network. The media data may be stored in a manner that is coordinated with any relevant metadata to facilitate identification and matching of media data and its associated metadata, for example video of a medical procedure on a patient and any metadata associated with that patient. The result of this conversion and coordinated storage of data collected by various source devices 102 is a central database maintained by the hospital data centre 104 of the system 100. This database brings together media data and metadata from a variety of different source devices 102 (which may each collect data in a variety of different formats) into a single data structure that can be managed and accessed centrally. The database may also maintain associations between media data and relevant metadata, as discussed in more detail below. The user devices 108 are also connected to the hospital data centre 104 and can access the data through an appropriate user interface, via which any of the stored data held by the central hospital data centre 104 may be accessed in the unified format. On receipt of any request for stored data (e.g. a download request made via the user interface), the hospital data centre 104 accesses the data and sends the data to the user device 108. As all data is stored in a unified format, the hospital data centre 104 can seamlessly access and route the data to the user regardless of what specific data is requested. The connection between the various components of the system shown in figure 1 may be provided by suitable cabling and/or connections over a local network or via wired and/or wireless internet connection.

### DATA MANAGEMENT

Figure 2 shows more details of the data management system 200 corresponding to the hospital data centre 104 of figure 1. In the embodiment of figure 1, data management system 200 is one component of the overall OR data control system 100, i.e. hospital data centre 104. However, as noted above, in other embodiments, the OR data control system may comprise just the data management system 200, and in such cases may be connected to one or more components, such as user devices, data sources (e.g. OR medical devices) and/or one or more remote databases. For example, the OR data control system of such an embodiment may take the place of the hospital data centre 104 of figure 1, connected to all the other various components shown in figure 1.

The data management system 200 comprises one or more media adaptors, which here comprises one or more image, video and or audio data set adaptors 202, and one or more metadata adaptors 204. The data management system 200 further comprises a core unit 206, also referred to as a communication unit herein, comprising one or more memory units 208. In this embodiment, the core unit 206 comprises one memory unit 208 for all of the image, video and or audio data, although it will be appreciated that separate storage units may be used. The core unit 206 further comprises a media management system 210, and a patient management system 212, each of which store any received metadata. The media management system 210 and patient management system 212 have been shown as separate units, but in other embodiments may be a single unit. It will also be appreciated that in some embodiments the communication unit 206 does not itself comprise any memory units (the memory unit 208, or a media management system 210, and a patient management system 212). In such embodiments, the communication unit may be communicatively coupled to separate memory units, and performs storage management and data access functions - i.e. sending received data for data storage, accessing requested data, and providing users that data. Here, the data management system (i.e. the MAM) may be a software solution comprising the adaptors and the communication unit, which are in communication with separate memory units.

The one or more adaptors 202, 204 are configured to receive two or more data sets, each data set received from a source device, here different source devices (e.g. a medical device such as medical cameras, endoscopes, MRI, X-Ray etc.), and each set having a different data format; convert the data format of each data set to a unified format; and send the converted data sets to the communication unit 206 (e.g. units 208, 210 and 212 of figure 2) for data storage. In this embodiment, a source device interfaces with a specific adaptor - the source device sends its data to the adaptor in a data stream. It should also be noted that we are using "data" broadly to include both media data such as videos, pictures audio etc., and metadata providing additional information about the patient, the medical procedure and/or the source device itself. Metadata can include, for example, patient data such as medical history; previously collected health data such as vital sign data, blood pressure etc.; information about the medical procedure such as the surgery being performed, timings, vital sign information, etc.; information about the source device such as device model; information about the media data being sent, etc. As discussed above, the term "data set" is being used simply to mean a group of associated data of the same general type (media data or metadata) and the same format. A data set may comprise media data in the form of video, audio and/or image data. For example, a data set may be a video file comprising video data of one or more frames of video data having a certain format. An audio data set may be an audio file comprising audio data having a certain format. Video data may have corresponding audio data (i.e. audio-video data) and the adaptors will generally be arranged to handle such audio-video data together. For clarity of illustration, discussion will focus on conversion of the video data, but it will be appreciated that any related audio will be appropriately converted in parallel, as will be clear to one skilled in the art. However, it will be appreciated that in some cases, video and audio can be handled separately, for example in the case of a camera without a microphone or a microphone without a camera. The system may be set up to handle any such cases. A data set in relation to image data may be one or more images of a single format, and a metadata set may be text data relating to a patient or a procedure, etc. Conversion of data sets from one format to another is being used in its common and well understood meaning, although keeping in mind that the specific details of the conversion will depend on the type of data being converted, as noted above. For example, conversion of different media data sets of different formats may be a conversion of video files having different formats into a unified format. Such a conversion may relate to the processing of videos to obtain the same video encoding and the same subset of resolutions and bitrates. Similarly, conversion of pictures to a unified format relates to processing images of different formats to obtain the same resolutions and to store them with the same extension. Similar considerations apply to audio data. The technical details of exactly how to perform a conversion from one format to another, for media data (video, audio and/or image) or metadata, would be clear to the skilled person and will not be discussed in further detail.

As shown in figure 2, the one or more media adaptors 202 comprise five adaptors - media adaptor 1, media adaptor 2, media adaptor 3, media adaptor 4 and media adaptor 5. Each adaptor 202 is configured to receive a corresponding set of image, video and/or audio media data as a separate media data stream, the streams indicated generally by numeral 214 in figure 2, with each stream being indicated by a solid arrow pointing at its corresponding adaptor. Each adaptor accepts its corresponding stream according to a certain communication protocol over which the media data is transported. As an example of the communication protocols, media adaptor 1 may receive a media data stream according to TCP, media adaptor 2 may receive a media data stream according to RTSP, media adaptor 3 may receive a media data stream according to RTP, and media adaptor 4 and 5 may receive a stream according to API or any other suitable communication protocol. Each media data stream carries as its payload media data having a certain format. Each data stream is received from a different device in one or more ORs, with the format of the stream being set by the format the corresponding OR device is configured to use. In the example of figure 2, each media adaptor is configured to process a different source format, so media adaptors 1, 2, 3, 4 and 5 respectively receive data of first, second, third, fourth and fifth formats. Possible formats may include MPEG2, H.264, MOV, WMV, AVI, AVCHD etc., for video, and OGG, MP3, AAC for audio, and any appropriate combinations are possible. For example, media adaptors 1 to 3 may utilise OGG format for audio and have different video formats as appropriate depending on the source devices in the OR. Similarly, if a given medical camera is configured to generate MPEG2 encoded files with MP3 audio, the system may be configured so that the medical camera sends its MPEG2 encoded file with MP3 audio to the corresponding adaptor, for example media adaptor 4, configured to process MPEG2 encoded file with MP3 audio. If another medical device is configured to generate H.264/AVC data with AAC audio, this may be sent to media adaptor 5, configured to process H.264/AVC data with AAC audio. In other words, the arrangement shown in figure 2 comprises five media data adaptors configured to receive five separate sets of video data (with any associated audio), each data set received from a separate source device in a separate data stream, based on a different protocol (e.g. TCP, RTSP, RTP) and having a different data format (e.g., MPEG2, H.264, MOV, WMV, AVI, AVCHD etc., for video, and OGG, MP3, AAC for audio). Each adaptor is configured to convert its respective data set into the unified format of the system 200, here H.264 with CMAF and associated audio of appropriate format (e.g. AAC). As discussed previously, each adaptor is configured to apply a conversion specific to the format of the data set it receives, and so the conversion of each adaptor is different (one adaptor's conversion is from MPEG2 to unified, another's is from a different format to unified, etc.). As noted above, the specific details of how to perform each individual conversion would be clear to one skilled in the art, and any appropriate source format may be handled by the system by the appropriate adaptor. It will be appreciated that this number and combination of adaptors is for illustration purposes, and any number or type of media adaptor may be used as appropriate for the use case (for example, two or more adaptors may be used, each receiving media data of a different format and according to a different communication protocol and converting the media data into the unified format). It will also be appreciated that any appropriate data format or codec conversion may be provided by an appropriate adaptor. In other words, the above referenced formats and protocols are one example used for illustration, but data sets of any other format, codec or protocol can be handled by the system by utilising the appropriate adaptors to receive data according to any appropriate protocol and convert any source formats into the unified format. For example, two or more of the adaptors may use the same communication protocol, but be configured to convert different data formats into the unified format.

Each adaptor 202 may also be configured to receive metadata that is associated with the media content received by that adaptor. For example, if an adaptor 202 is receiving a video and audio stream is from a medical camera, the adaptor may also receive metadata providing further details regarding that video and audio stream, for example, time stamps, camera location, device type, as well as additional medical information. Although the present embodiment shows separate adaptors for each received data set, it will be appreciated that a single adaptor may be used for all sets, or that a variety of adaptors are used, some receiving more than one data set and others receiving only a single set. It will also be appreciated that more than one set may be received from a single source device, e.g. multiple media data streams or a media stream and a metadata stream. It is also noted that the source devices will generally be located in the operating room, but external source devices are also contemplated. For example, the MAM may receive data sets from other IT systems (computers, laptops etc.) to supplement the information being received from the operating room, for example additional patient metadata being received from a computing device outside of the operating room.

The one or more metadata adaptors 204 similarly comprise four adaptors - metadata adaptor 1, metadata adaptor 2, metadata adaptor 3 and metadata adaptor 4. Similar to the media data adaptors, the metadata adaptors receive metadata of certain formats according to appropriate protocols. For example, the metadata adaptors may receive metadata according to the gRPC API or REST API protocol, e.g. metadata adaptors 2 and 3 may be configured to respectively receive metadata according to gRPC API and the REST API, with metadata adaptors 1 and 4 using other appropriate protocols or APIs. Each metadata adaptor is configured to receive metadata of a certain format, so metadata adaptors 1, 2, 3 and 4 respectively receive metadata of first, second, third, and fourth formats. Examples of possible metadata formats include XML or HL7 format, but it will be appreciated that any appropriate format is contemplated. For example, metadata adaptor 1 may receive XML files containing metadata, metadata adaptor 4 is configured to receive the HL7 formatted metadata, with metadata adaptors 2 and 3 configured to receive metadata of other appropriate formats. Each adaptor 204 is configured to receive a corresponding set of metadata as a separate metadata stream 216. This metadata may comprise further media metadata relating to the media data sets received by the one or more media adaptors 202 and/or patient metadata providing patient data relevant to the media data being received. For example, if video and audio data is being received from a camera in an operating room during an operation on a patient, patient data, such as age, weight, health record data, vital sign information, may be concurrently received. As with the media adaptors, it will be appreciated that this number and combination of adaptors is for illustration purposes, and any number or type of media adaptor may be used as appropriate for the use case. The above referenced formats and protocols are one example used for illustration, but metadata sets of any other format, codec or protocol can be handled by the system by utilising the appropriate adaptors to receive metadata according to any appropriate protocol and convert any source formats into the unified format.

The adaptors receiving this corresponding media data and metadata (which may be the same adaptor or different adaptors) may attach a common identifier, ID to the media data and the corresponding metadata. The way the adaptors identify corresponding data sets and attach common IDs depends on the type of adaptor involved. In general, the overall system is able to recognise matching data sets in view of the protocol regulating each adaptor's behaviour. As a specific example, if an adaptor receives metadata via XML file, the system is able to identify the patient data because the incoming data are listed against patient related tags. (E.g. <patient-name>Jason</patient-name>), which is to say, the adaptor is able to identify a patient for a particular incoming data set by processing a patient tag attached to the incoming data. The adaptor can interpret this tag from the incoming metadata and assign the converted metadata an ID internal to the system that corresponds to that patient. If a second adaptor then receives e.g. media data corresponding to the same patient, this second adaptor can similarly identify the patient in light of patient tags within the received media data, and assign the converted media data the same internal ID for that patient. Thus, a coordinated ID system is established between the adaptors that links relevant data together. There may be various IDs employed, for example, relating to particular patients, medical procedures, operating rooms etc., and all the IDs may be stored as a matrix or database, which can then be used to identify related data and metadata within the system.

When the media data sets and metadata sets are received by each adaptor 202, 204, each adaptor converts the media data and metadata into a unified format, i.e. such that all the different types of media data and all the different types of metadata have the same unified format, which is to place them in the appropriate form for the database maintained by the system, or in other words, to translate the data into the native/internal language of the data management system so that the data can be stored, managed, accessed and generally processed by the system centrally, regardless of the source format of the data. The actual conversion of a given format to another format uses techniques known in the art and will be clear from the above discussion, and therefore will not be discussed further here. The point, however, is that each adapter, whether a media adapter or a metadata adapter, and regardless of the format of the original source data/metadata, converts the data into the unified format. There is coordination between all adaptors to provide a single format for every piece of media data and metadata received by the data management system 200.

Once the data sets and metadata have been received and converted into the unified format of the OR data system 100, the adaptors are configured to send the converted data sets to the communication unit 206 for storage in one or more of the one or more memory units. Storage management (along with data access) is performed by processing unit 218 of the communication unit. As shown in figure 2, the communication unit 206 sends the converted media data from media adaptors 202, that is image, video and/or audio data, to storage unit 208. The communication unit 206 sends any associated metadata (also converted to the unified format) from media adaptors 202 to the media management system 210. Converted media metadata from the metadata adaptors 204 are also sent to the media management system 210, and converted patient metadata are sent to the patient management system 212. The routing pathways of the converted data/metadata from the adaptors to their respective storage units are shown in figure 2 by arrows running from each adaptor to the storage unit 208, the media management system 210 and the patient management system 212, and are managed by the storage management function of the processing unit 218 of communication unit 206. The communication unit is thus configured to send the converted data sets (the media sets) to the same memory unit and each converted metadata set to one or more memory units different to the memory unit of the converted data sets. It will be appreciated, however, that different storage arrangements may be used, for example one memory unit for all metadata, and one memory unit for all media data. Generally, the media data and the metadata will be stored in separate storage, although they may be stored together in a single storage. In either case, the storage of the media data and the metadata is coordinated to allow retrieval of related data. For example, the data may be stored according to the IDs of the data in question. In particular, a matrix or database, e.g. as discussed above, may associate a particular memory location (a bucket, folder etc.) with an ID, and any media data assigned by an adaptor to that ID may be stored at that location (in one or more files, for example). Relevant metadata e.g. for a patient, may also be maintained, for example in separate storage, and may also be associated with the ID. Thus, both the media data set and the metadata set may be identified (by ID) and accessed in parallel to present the media data and related metadata, for example for a patient, procedure, OR etc., on request.

In this way, the data management system 200 may centrally receive all media data and metadata being generated from medical devices in use in ORs across the hospital and seamlessly convert all that data into a unified format in a coordinated fashion. Further, as the reception and conversion is centralised and coordinated, the storage of the data and the metadata can also be performed in a coordinated fashion, in particular, by storing data alongside their internally assigned IDs that establish correspondence with other related data or metadata stored within the system. In other words, media data and its associated metadata and patient data may be received together, and thus may be stored in a coordinated fashion so as to facilitate identification and access of media data and associated metadata.

It is noted that the processing unit 218 is communicatively coupled to the one or more memory units 208, 210 and 212. The processing unit 218 may comprise one or more processors configured to perform the functions of the communication unit 206 discussed herein, such as storage management and data access (i.e. the processing unit 218 may further comprise one or more data access units to access the memory units).

As described in relation to figure 2, adaptors 202 and 204 receive data sets and metadata sets via the data streams 214 and 216, convert the data and the metadata to a unified format and then send the converted data and metadata to core unit 206 for it to be stored in the one of more memory units, e.g. storage unit 208, media management system 210 and patient management system 212. Once the media data and the metadata have been stored, this data and metadata may be accessed by processing unit 218, which is to say the processing unit is communicatively coupled to the one or more memory units 304. The processing unit 218 is configured to provide user devices, such as user devices 108 of figure 1, the stored data and metadata. For example, a user may use a user interface to request stored data. Responsive to receiving the request, the processing unit retrieves the requested data/metadata and routes the requested stored data to the user device. This may be achieved by the processing unit 218 processing parameters of the user request to identify the desired data and then querying the memory units 208, 210 and 212 to retrieve the desired data and provide this to the user. As all media data is stored centrally in a unified format, all the data, and all the metadata, is accessible to the user in a single format, regardless of how many different source devices were used in collecting the data. The user may request any type of data with a single request, regardless of the type or format of the data as originally generated. In other words, the user device, does not have to communicate with multiple different databases of different formats, each requiring separate communication protocols and request message formats, which puts a large communication burden on the user devices communicating separately with different entities in order to obtain all the desired data. Rather, a single request may be sent to the data management system 200 and the request data is seamlessly provided to the user.

Similar considerations apply to stored metadata. In particular, the processing unit 218 may be configured to receive from a user device 108 a request for stored data and stored metadata corresponding to the requested stored data; and responsive to receiving the request, routing the requested stored data and metadata to the user device. For example, a user may request, in a single request, video of a medical procedure captured by a camera of a medical device, and any media and patient metadata associated with the video and the patient of the procedure. As all the data has been centralised and stored in a coordinated manner by the data management server 200, the processing unit 218 can seamlessly locate and retrieve the requested video and its associated metadata and provide this to the user. For example, the processing unit may make use of the matrix of IDs to locate the stored media data and its corresponding metadata.

In centralising the storage of media data and metadata in a coordinated manner, the ability of the system to quickly and reliably identify and access metadata that is relevant to a requested piece of media content is improved. In particular, the data control system is able to convert all data and metadata into a unified format and then store both appropriately and in a way that allows easy identification of the metadata when given media content is requested by a user. This has been discussed above, where both a media set and its corresponding metadata are converted into a unified format, and an association is formed between the media data and the metadata that links the media data to the storage location of the metadata. Further, the position of the database management system 200 on the network allows the communication unit 218 to act as the central hub for all user devices. This results in a standardisation of format across the hospital, and allows users to interact with a single entity for all requests for data.

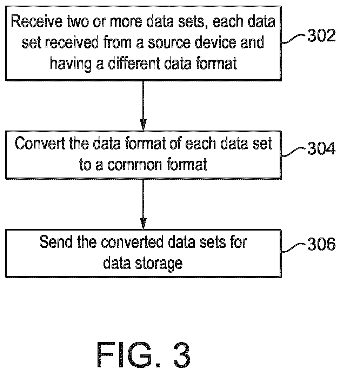

In use, the data control system performs the method shown in the flow diagrams of figure 3. The method comprises receiving 302 two or more data sets, each data set received from a source device, e.g. a different source device of an operating room, and having a different data format; converting 304 the data format of each data set to a unified format; and sending 306 the converted data sets for data storage, e.g. to one or more of the one or more memory units. The details of these steps have been discussed above in relation to figures 1 and 2.

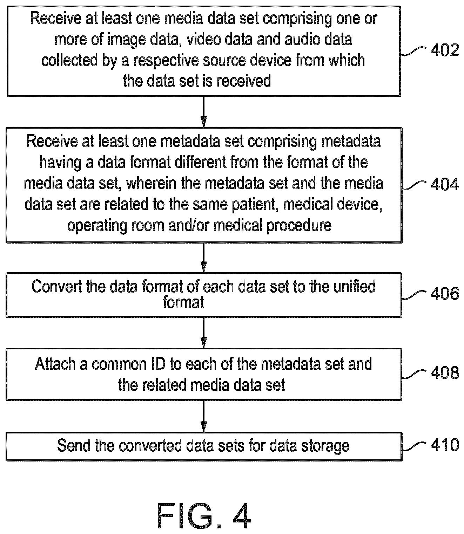

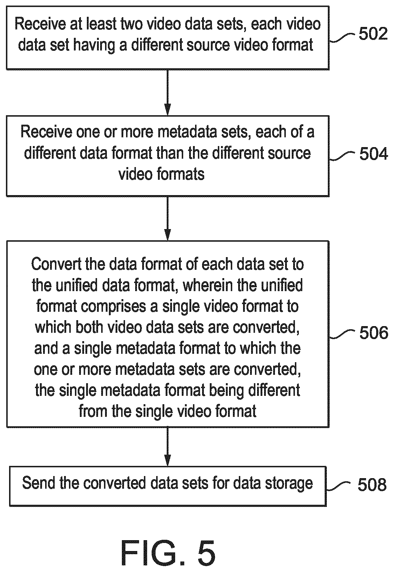

Figures 4 and 5 illustrates further details of the method of figure 3, according to one or more embodiments of the disclosure. These details may be used alone or in combination. As shown in figures 4 and 5, the first step 302 of receiving the two or more data sets may comprise steps 402 and 404 of figure 4, which comprise receiving 402 at least one media data set comprising one or more of image data, video data and audio data collected by a respective source device from which the data set is received, and receiving 404 at least one metadata set comprising metadata having a data format different from the format of the media data set, wherein the metadata set and the media data set are related to the same patient, medical device, operating room and/or medical procedure.

As shown in figure 5, receiving the at least one media data set may comprise the step 502 of receiving at least two video data sets, each video data set having a different source video format (for example, each may have any of those video formats listed above with respect to figure 2, e.g. one video data set having MPEG2, and the other having H.264), and receiving the at least one metadata set may comprise the step 504 of receiving receive one or more metadata sets, each of a different data format (e.g. XML, HL7, etc.) than the different source video formats. The at least two video data sets may be received in separate data streams according to one or more communication protocols. As one example, the at least two video data sets may be received in separate data streams by separate adaptors according to different communication protocols. As shown in figure 2, the adaptors 202 and 204 may receive the various data sets of different formats. In this case, the adaptors 202 receive the five streams of media data 214 of differing formats alongside associated metadata and the adaptors 204 receive the four streams of metadata 216 of different formats. In the embodiment shown in figure 2, each stream (carrying one or more data sets) is received by a separate adaptor, and the streams may be received according to one or more different communication protocols, as discussed in more detail above.

The adaptors 202 and 204 at step 304 convert each of their received data sets into a unified format, i.e. into the central operating format of the overall system. This is also shown as step 406 in figure 4. As shown at step 506 of figure 5, the unified format may comprise a single video format to which the at least two video data sets are converted, and a single metadata format to which the one or more metadata sets are converted, the single metadata format being different from the single video format. In the embodiment of figure 2, for example, the at least two video data sets comprise five video data sets all being converted into the single video format. As discussed elsewhere, each different source format has its own distinct conversion into the unified format. For example, the method may involves a first conversion of video of a first format into the unified format, a second conversion of video of a second format into the unified format, etc., with each adaptor configured to perform the conversion specific to the format of the data set it receives. This may be done on the fly as the data is received, and in parallel - i.e. each adaptor performing its conversion in parallel with one another.

In some embodiments, the adaptors may then attach a common ID to each of the metadata set and the related media data set, as shown by step 408 of figure 4. In more detail, this may involve receiving a metadata set for a corresponding media data set of the two or more data set. For example, the stream received by media adaptor 1 may carry metadata relating to the media data of the stream, or one of the separate metadata streams 216 may carry patient data corresponding to the media data carried by the stream received by media adaptor 1. The adaptor(s) receiving the stream(s) may associate the metadata set and the corresponding media data set with a common ID; the metadata set and the corresponding media data set will thus be associated with a common ID in storage. In other words, the method may comprise attaching the common ID to each of the metadata set and the corresponding media data set before sending the metadata set and the corresponding media data set to the communication unit for storage. The metadata set and its corresponding media data set may be related to the same patient, the same operating room and/or the same medical procedure. The metadata set may comprise one or more of image, video, audio, source device, medical procedure or patient metadata.

The converted data is then sent at step 306 (step 410 of figure 4, or 508 of figure 5) by the adaptors to the communication unit 206 for storage. The communication unit 206 then stores the converted data sets. This step may be performed by processing unit 218, which may send the converted data sets to one or more memory units 208, 210, and 212, for storage. The processing unit 218 determines the pathways from the adaptors to storage, ensuring that converted media data is stored in media storage 208, patient metadata is stored in storage 212, and other metadata, for example non-patient metadata relating to the received media data, in storage 210.

As discussed above with respect to figure 2, the system may be configured so that multiple media data sets and metadata sets may be concurrently received, converted and sent to storage. The method may further comprise sending the converted media data set to the same memory unit 208 and each converted metadata set to one or more memory units 210, 212, different to the memory unit of the converted media data set. The method may further comprise providing stored data in the unified format to one or more user devices, in particular, the processing unit 218 may receive requests for data from user devices and access the necessary memory units to provide the data, as described above. Each source device may be located in the operating room, or one or more source devices may be located in the operating room and one or more other source devices may be located outside the operating room.

### FURTHER EXAMPLES AND EMBODIMENTS OF THE PRESENT DISCLOSURE

Although the above arrangements have been described in relation to the conversion of two or more data sets, each data set having a different data format, it will be appreciated that arrangements are possible in which there is only a single data set being converted into the unified format. In such an arrangement there may be a single adaptor configured to receive a data set, convert the data set into the unified format and send the converted data set for storage.

In the illustrated embodiments some components may be integrated at a circuit, package or die level.

In the illustrated embodiments of the disclosure the system may comprise a computing and/or electronic device.

Such a device may comprise one or more processors which may be microprocessors, controllers or any other suitable type of processors for processing computer executable instructions to control the operation of the device in order to gather and record routing information. In some examples, for example where a system on a chip architecture is used, the processors may include one or more fixed function blocks (also referred to as accelerators) which implement a part of the method in hardware (rather than software or firmware). Platform software comprising an operating system or any other suitable platform software may be provided at the computing-based device to enable application software to be executed on the device.

The computer executable instructions may be provided using any computer-readable media that is accessible by computing based device. Computer-readable media may include, for example, computer storage media such as a memory and communications media. Computer storage media, such as a memory, includes volatile and non-volatile, removable and non-removable media implemented in any method or technology for storage of information such as computer readable instructions, data structures, program modules or other data. Computer storage media includes, but is not limited to, RAM, ROM, EPROM,

EEPROM, flash memory or other memory technology, CD-ROM, digital versatile disks (DVD) or other optical storage, magnetic cassettes, magnetic tape, magnetic disk storage or other magnetic storage devices, or any other non-transmission medium that can be used to store information for access by a computing device. In contrast, communication media may embody computer readable instructions, data structures, program modules, or other data in a modulated data signal, such as a carrier wave, or other transport mechanism. As defined herein, computer storage media does not include communication media.

Any range or device value given herein may be extended or altered without losing the effect sought, as will be apparent to the skilled person.

It will be understood that the benefits and advantages described above may relate to one embodiment or may relate to several embodiments. The embodiments are not limited to those that solve any or all of the stated problems or those that have any or all of the stated benefits and advantages.

Any reference to 'an' item refers to one or more of those items. The term 'comprising' is used herein to mean including the method steps or elements identified, but that such steps or elements do not comprise an exclusive list and a method or apparatus may contain additional steps or elements.

The order of the steps of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

It will be understood that the above description of a preferred embodiment is given by way of example only and that various modifications may be made by those skilled in the art. Although various embodiments have been described above with a certain degree of particularity, or with reference to one or more individual embodiments, those skilled in the art could make numerous alterations to the disclosed embodiments without departing from the spirit or scope of this disclosure .

## Claims

1. An operating room data system for a medical facility comprising:
one or more adaptors; and
a communication unit;
wherein the one or more adaptors are configured to:
receive two or more data sets, each data set received from a source device and having a different data format;
convert the data format of each data set to a unified format; and
send the converted data set to the communication unit for data storage.

2. The operating room data system of claim 1, wherein the communication unit is configured to send the converted data sets to one or more memory units for storage.

3. The operating room data system of claim 1 or 2, wherein at least one of the two or more data sets is a media data set comprising one or more of image data, video data and audio data collected by a respective source device from which the data set is received.

4. The operating room data system of claim 3, wherein at least one other of the two or more data sets is a metadata set comprising metadata having a data format different from the format of the media data set.

5. The operating room data system of claim 4, wherein the one or more adaptors are further configured to receive a metadata set for a corresponding media data set of the two or more data set.

6. The operating room data system of claim 5, wherein the metadata set and the corresponding media data set are associated with a common ID in storage.

7. The operating room data system of claim 6, wherein the one or more adaptors are configured to attach the common ID to each of the metadata set and the corresponding media data set before sending the metadata set and the corresponding media data set to the communication unit for storage.

8. The operating room data system of any one of claims 4 to 6, wherein the metadata set and its corresponding media data set are related to the same patient, the same operating room and/or the same medical procedure.

9. The operating room data system of any one of claims 4 to 8, wherein the metadata set comprises one or more of image, video, audio, source device, medical procedure and/or patient metadata.

10. The operating room control system of any of claims 4 to 9, wherein the communication unit is configured to send converted media data set to the same memory unit and each converted metadata set to one or more memory units different to the memory unit of the converted media data set.

11. The operating room data system of any preceding claim, wherein the communication unit is configured to provide stored data in the unified format to one or more user devices.

12. The operating room data system of any preceding claim, wherein:
the communication unit comprises one or more memory units and one or more data access units for retrieving stored data from the one or more memory units;
the one or more adaptors comprise one adaptor for each received data set; and/or
each source device is located in the operating room, or one or more source devices are located in the operating room and one or more other source devices are located outside the operating room.

13. The operating room data system of any preceding claim, wherein at least two of the two or more data sets are video data sets comprising video data, each video data set having a different source video format, wherein the unified format comprises a single video format to which both video data sets are converted.

14. The operating room data system of claim 13, wherein the two or more data sets further comprise one or more metadata sets each of a different data format than the different source video formats, wherein the unified format further comprises a single metadata format to which the one or more metadata sets are converted, the single metadata format being different than the single video format.

15. A method of adapting operating room data, the method comprising:
receiving two or more data sets, each data set received from a source device and having a different data format;
converting the data format of each data set to a unified format; and
sending the converted data set for data storage.
